# EUROPÄISCHE PATENTSCHRIFT

(11) **EP 1 475 031 B1**
(45) Veröffentlichungstag und Bekanntmachung des Hinweises auf die Patenterteilung: **24.03.2010**
(21) Anmeldenummer: 04010501.7
(22) Anmeldetag: 03.05.2004
(51) Int. Cl.: A61B 1/012

(54) **Endoskopschaft**
Endoscope shaft
Tige endoscopique

(30) Priorität: 05.05.2003 DE 10320228
(43) Veröffentlichungstag der Anmeldung: 10.11.2004
(73) Patentinhaber: Invendo Medical GmbH, 69469 Weinheim (DE)
(72) Erfinder: Bob, Konstantin, Dr., 69469 Weinheim (DE); Kast, Wolfgang, 86420 Diedorf (DE)
(74) Vertreter: Winter, Brandl, Fürniss, Hübner Röss, Kaiser, Polte Partnerschaft Patent- und Rechtsanwaltskanzlei

(56) Entgegenhaltungen:
- WO-A-99/51283
- DE-A- 3 712 643
- DE-A- 4 413 026
- US-A- 4 805 595
- US-A- 4 900 314
- US-A- 4 928 699
- PATENT ABSTRACTS OF JAPAN Bd. 1995, Nr. 10, 30. November 1995 (1995-11-30) & JP 07 184828 A (OLYMPUS OPTICAL CO LTD), 25. Juli 1995 (1995-07-25)

## Beschreibung

Die vorliegende Erfindung betrifft einen Endoskopschaft gemäß dem Oberbegriff des Patentanspruchs 1.

Endoskope sind Geräte, insbesondere zur Exploration von Hohlräumen oder röhrenartigen Kanälen des Körpers beispielsweise für medizinische Zwecke. Derartige Endoskope werden vorzugweise zur Exploration der Speiseröhre, des Magens, des Zwölffingerdarms vom Magen aus, des Darms vom Anus aus, der Harnröhre, der Blase und der Harnleiter verwendet. Zumeist sind Endoskope an ihrem Vorderende mit einer Beleuchtungseinrichtung und mit einer Optik zur visuellen Erfassung des davor liegenden Bereichs des Körperhohlraums oder Körperkanals ausgerüstet.

Endoskope weisen ferner in der Regel einen sog. Arbeitskanal auf, durch den diverse Arbeitsinstrumente eingeführt und bedient werden können, z.B. Zangen zur Entnahme von Gewebeproben, Biopsienadeln, beheizbare Schneiddrähte, kleinere Scheren oder dergleichen. Schließlich sind in der Regel Funktionskanäle beispielsweise ein Fluidkanal für Spülflüssigkeit und Bedienungsdrähte zum Abwinkeln des Endoskopvorderendes in mehrere Richtungen vorhanden. Insgesamt hat das Endoskop, abgesehen von seinem hinteren Bedienungsende und einem Anschlußstrang, eine langgestreckte, biegsame stab- bzw. schaftförmige Gestalt. Übliche Außendurchmesser liegen etwa im Bereich von 9 bis 15 mm, am vorderen Kopf etwas größer.

Bisher werden Endoskope dadurch in den Körper eingeführt, dass der Arzt von dem aus dem Körper herausragenden Teil des Endoskops her das drucksteife Endoskop bzw. den drucksteifen Endoskopschaft in den Körper hinein schiebt. Diese Art des Einführens des Endoskops ist besonders mühsam, schwierig und auch für den Patienten insbesondere beim Koloskop äußerst schmerzhaft, da im letzteren Fall der Darm Abbiegungen und häufig Engstellen aufweist, denen der Endoskopschaft folgen muß. Demzufolge gehören koloskopische Untersuchungen bisher zu den aufwendigen und für den Patienten unangenehmen und schmerzhaften Untersuchungen. Das Umgehen mit einem Koloskop erfordert zudem einen entsprechend erfahrenen Arzt.

Des weiteren hat sich aus dem Stand der Technik gezeigt, dass Endoskope der bekannten Bauart aufgrund der von ihnen geforderten Steifigkeit für ein Einschieben in den zu untersuchenden Patientenhohlraum, bei gleichzeitiger Biegefähigkeit äußerst aufwendige und damit kostenintensive Konstruktionen darstellen. Diese Konstruktionen sind derart teuer, dass sie immer wieder verwendet müssen. Es ist daher notwendig, nach jeder Untersuchung aufwendige Reinigungs- und Hygienemaßnahmen durchzuführen, wobei letztlich auch eine Gefahr einer Beschädigung des Endoskopschafts besteht, insbesondere dann, wenn ungeschultes Personal derartige Reinigungsvorgänge durchführt. Darüber hinaus sind die erzielbaren Biegeradien so groß, dass eine im wesentlichen schmerzfreie Untersuchung beispielsweise einer Darmwand nicht möglich ist.

Angesichts dieser Sachlage besteht die Aufgabe der Erfindung darin, einen Endoskopschaft zu schaffen, der bessere Eigenschaften beipielsweise bezüglich seiner Flexibilität aufweist.

Diese Aufgabe wird durch einen Endoskopschaft mit Merkmalen des Anspruchs 1 gelöst.

Der Kern der Erfindung besteht demzufolge im wesentlichen darin, den Endoskopschaft mit einem Arbeitskanal und einer Anzahl von Versorgungs- und/oder Funktionskanälen auszubilden, welche im Endoskopschaft so angeordnet sind, dass sie sich aufgrund deren Anordnung in Schaftlängsrichtung längen können.

Dies wird im Prinzip dadurch erreicht, dass die Kanäle eine Überlänge bezüglich des Endoskopschafts erhalten. Auf diese Weise wird das Biegeverhalten weniger durch die Versorgungs- und/oder Funktionskanäle beeinträchtigt. Darüber hinaus kann der Endoskopschaft nunmehr gebogen werden, ohne dass das Risiko eines Einreißens von Kanalwänden entsteht.

Die DE 44 13 026 A1 und die JP 7 184 828 A offenbaren einen Endoskopschaft gemäß dem Oberbegriff des Patentanspruchs 1.

Erfindungsgemäß sind der/die Versorgungs- und/oder Funktionskanäle, welche in der Regel außermittig im Endoskopschaft liegen, spiralförmig um den Arbeitkanal verlaufend angeordnet. Auf diese Weise ist die Flexibilität des Endoskopschafts weniger von den Eigenschaften der für die Kanäle verwendeten Materialien abhängig sondern erhält seine Biegefähigkeit durch die erfindungsgemäße Formgebung bzw. Anordnung der Kanäle als Wendel. Mit dieser Konstruktion lassen sich Biegedurchmesser von weniger als 30mm erreichen, ohne dass ein Aufbrechen der Kanalwände auftritt.

Hierfür sind die Versorgungs- und/oder Funktionskanäle sowie vorzugsweise auch die Kabel oder Fasern vorab zu einem Flachband verbunden, welches spiralförmig aufgewickelt ist und dessen eine Flachseite gleichzeitig die Innenwand des Arbeitskanals bildet. An dieser Stelle sei jedoch darauf hingewiesen, das an Stelle des Flachbands natürlich auch andere Gestaltungsmöglichkeiten wie ein Kanal-Schlauchpaket und dergleichen bestehen.

Auch müssen die Versorgungskanäle bzw. Funktionskanäle nicht notwendiger Weise ein Rundprofil aufweisen, wie dies u.a. in einigen der nachfolgenden Ausführungsbeispielen beschrieben ist, sondern können jede beliebige Form annehmen, die geeignet ist, eine kompaktere Bauweise zu ermöglichen. So können die Kanäle beispielsweise eine Rechtecks- oder Dreiecksform annehmen und unmittelbar von dem Flachbandmaterial selbst, beispielsweise durch Strangpreßformen gebildet sein.

Auch hat es sich als vorteilhaft erwiesen, den Endoskopschaft aus einem Kunstharzschaum zu fertigen, im dem die Versorgungsund Funktionskanäle eingebettet sind. Ein derartigen Schaummaterial bildet an seiner Oberfläche, d.h. der Mantelfläche des Endoskopschafts Poren, Vertiefungen und dergleichen Oberflächenunebenheiten, die bei einem Abbiegen des Endoskopschafts eine Faltenbalgwirkung hervorrufen, d.h. die Oberflächenspannung infolge von Auseinanderziehen oder Zusammenfalten von Oberflächenfalten reduzieren.

Weitere vorteilhafte Ausgestaltungen der Erfindung sind Gegenstand der übrigen Unteransprüche.

Die Erfindung wird nachstehend anhand bevorzugter Ausführungsbeispiele unter Bezugnahme auf die begleitenden Zeichnungen näher erläutert.

Es zeigen:
- Fig. 1: die teilweise aufgebrochene Funktionsansicht eines Endoskopschafts mit Arbeitskanal und spiralförmig angeordneten einzelnen Versorgungs- und/oder Funktionskanälen, der jedoch nicht Gegenstand der Erfindung ist ,
- Fig. 1a: die Perspektivenansicht des Endoskopschafts gemäß Fig. 1,
- Fig. 2: die Funktionsansicht von spiralförmig angeordneten Versorgungs- und/oder Funktionskanälen gemäß einem ersten Ausführungsbeispiel der Erfindung, die zu einem Flachband zusammengefasst bzw. integriert sind,
- Fig. 3: die Perspektivenansicht eines Teils eines Endoskopschafts, der durch ein Flachband gemäß dem ersten bevorzugten Ausführungsbeispiel der Erfindung gebildet wird,
- Fig. 4: die Perspektivenansicht eines Flachbands gemäß einem zweiten Ausführungsbeispiel der Erfindung, und
- Fig. 5a, 5b: die Perspektivenansicht eines Kanal-Faser-Schlauchpakets gemäß einem dritten Ausführungsbeispiel der Erfindung.

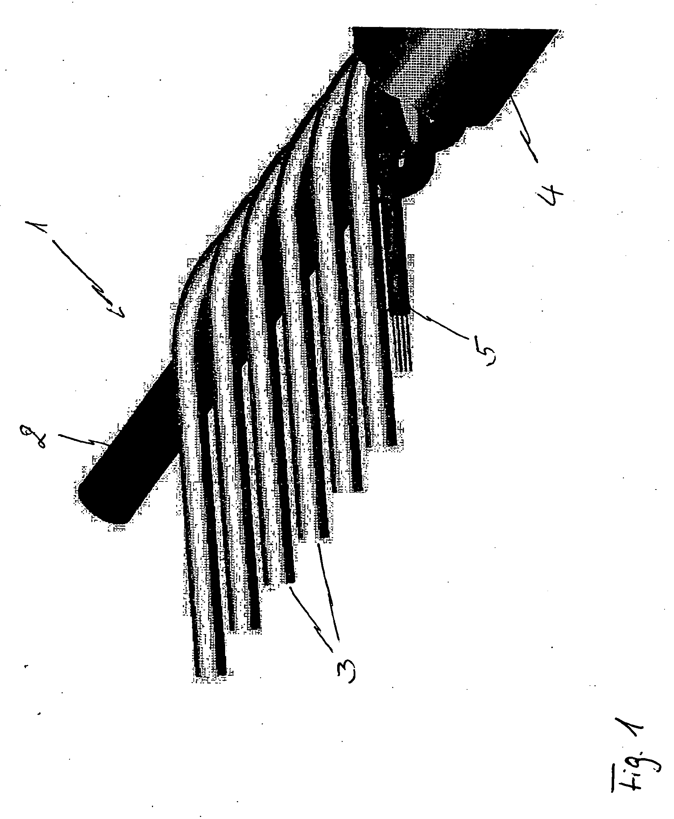

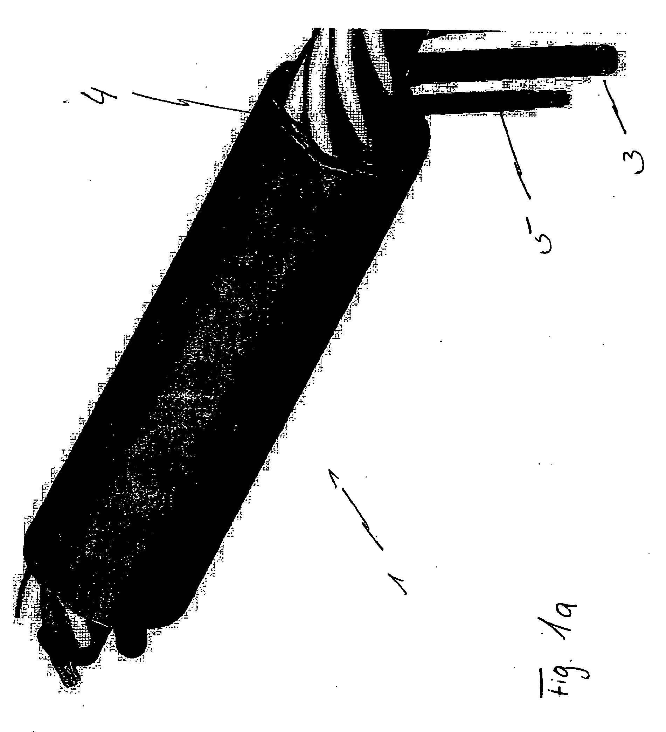

Wie insbesondere aus der Fig. 1 zu entnehmen ist, hat oder bildet ein Endoskopschaft 1 allgemein Arbeitskanal 2 sowie eine Anzahl von Versorgungs- und/oder Funktionskanälen 3. Die Versorgungs- und/oder Funktionskanäle 3 sind spiralförmig aufgewickelt und verlaufen um den im wesentlichen zentralen Arbeitkanal 2 herum.

Der so aufgebaute Endoskopschaft 1 hat in der Regel an seinem distalen Ende einen Endoskopkopf (nicht weiter dargestellt), der über die Versorgungskanäle 3 mit einer ebenfalls nicht gezeigten Betätigungseinrichtung verbunden ist, welche vorzugsweise am proximalen Ende des Endoskopschafts 1 angeordnet ist. Der Endoskopkopf hat eine Anzahl von einsatzspezifischen Funktionselementen wie eine Optik, Beleuchtungseinrichtungen, Sprühdüsen, bewegliche Linsen und/oder Spiegel und dergleichen, welche über die Versorgungskanäle 3 mittels der Betätigungseinrichtung bedienbar sind. Darüber hinaus ist der distale Endabschnitt des Endoskopschafts 1 vorzugsweise als ein abwinkelbares Gelenkstück ausgebildet, welches an den Funktionskanälen 3 angeschlossen und ebenfalls mittels der Betätigungseinrichtung z.B. hydraulisch einstell- bzw. verschwenkbar ist.

An dieser Stelle sei darauf hingewiesen, das die Konstruktion derartiger abwinkelbarer Endstücke für Endoskopschäfte sowie der Aufbau von Endoskopköpfen bereits zum Stand der Technik gehören und auch von der Erfinderin der vorliegenden Schaftkonstruktion selbst im Rahmen einer Vielzahl von Patentanmeldungen publiziert wurden. In sofern kann an dieser Stelle auf eine detailierte Beschreibung des Endoskopkopfs sowie des abwinkelbaren Endstücks verzichtet werden.

Gemäß der Fig. 1 wird der Arbeitskanal 2 von einem flexiblen Rohr oder Schlauch vorzugsweise aus einem Kunststoffmaterial gebildet, um den eine Hülle 4 vorzugsweise aus einem Kunstharzschaum geformt ist. Wie aus der Fig. 1a zu entnehmen ist, sind in dieser Hülle 4 die Versorgungs- und/oder Funktionskanäle 3 eingebettet, welche einzeln, in einem vorbestimmten Parallelabstand zueinander sowie vorzugsweise Winkelsymmetrisch um den Arbeitskanal 2 zu einer Spirale mit bestimmter Steigung gewunden sind. Des weiteren ist ein Faser- oder Kabelbündel 5 vorgesehen, welches parallel zu den Versorgungs- und/oder Funktionskanälen 3 ebenfalls um den Arbeitskanal 2 spiralförmig geführt und in die Hülle 4 eingebettet ist. Die Versorgungs- und/oder Funktionskanäle 3 wie auch das Kabelbündel 5 sind dabei jedoch in einem vorbestimmten Radialabstand zum Arbeitskanal 2 angeordnet, so dass sie vom Hüllenmaterial vollständig umschlossen sind. Hierdurch wird gewährleistet, dass die Innenwand des Arbeitskanals 2 auch bei maximaler Abkrümmung des Endoskopschafts 1 nicht von den umlaufenden Kanälen 3 oder Kabeln 5 eingedrückt wird. Die Versorgungs- und/oder Funktionskanäle 3 bestehend vorliegend aus einem Rohrprofil aus Kunststoff mit einer vorbestimmten Flexibilität, sodass die spiralförmig angeordneten Versorgungs- und/oder Funktionskanäle 3 ohne großen Widerstand auseinander gezogen werden können, ohne dass die Kanalwände brechen oder einreißen.

Eine alternative Ausgestaltung zu der vorstehend beschriebenen Ausführungsform besteht darin, den Arbeitskanal 2 nicht als ein separates Bauteil vorzusehen, sondern im Rahmen der Formgebung der Hülle 4 auszubilden. In anderen Worten ausgedrückt kann der rohrförmige Arbeitskanal 2 durch einen Dorn ersetzt werden, um welchen die Versorgungs- und/oder Funktionskanäle 3 sowie vorzugsweise das Faser- oder Kabelbündel 5 gewunden sind. Anschließend wird um den Dorn herum die Hülle 4 beispielsweise durch Schäumen, Extrudieren oder Gießen ausgebildet, wobei sich an der Oberfläche des Dorns eine glatte Innenwand ausbildet. Schließlich wird der Dorn aus dem ausgehärteten Hüllenmaterial gezogen, wodurch der Arbeitskanal 2, gebildet durch das Hüllenmaterial selbst, entsteht. Auch die Versorgungs- und/oder Funktionskanäle 3 können in einer entprechenden Weise gefertigt sein, d.h. von dem Hüllenmaterial selbst ausgebildet werden.

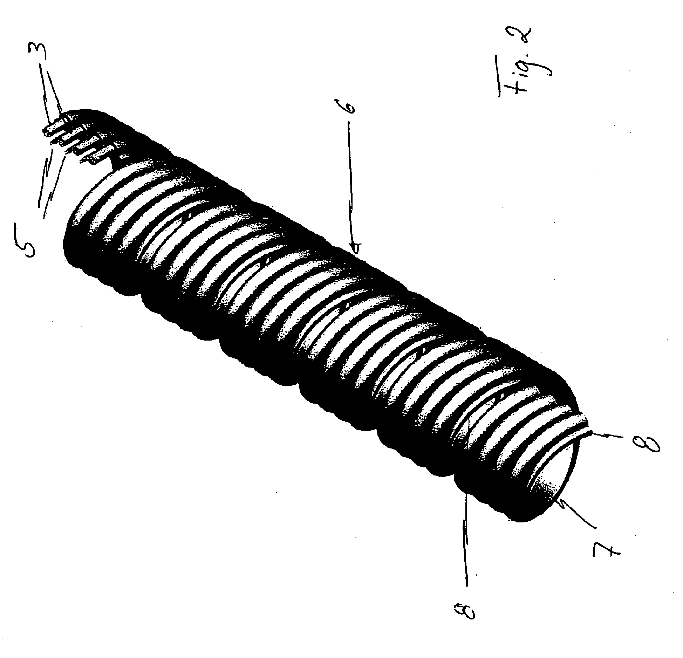

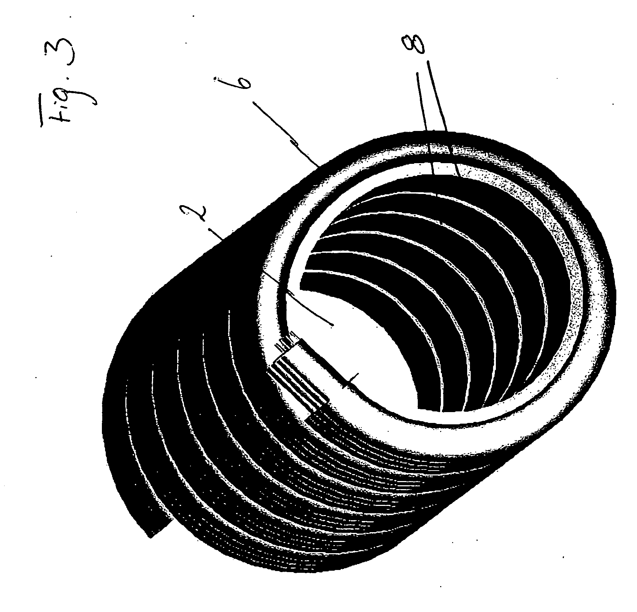

In der Fig. 2 und 3 ist nunmehr ein erstes Ausführungsbeispiel der Erfindung dargestellt, wobei im Nachfolgenden nur die Unterschiede zur Ausführungsform gemäß Fig.1 beschrieben werden. In diesem Fall sind die Versorgungs- und/oder Funktionskanäle 3 sowie vorzugsweise auch die Kabel und/oder Fasern 5 zu einem Flachband 6 zusammengefasst. D.h., die Kanäle 3 und ggf. die Kabel und Fasern 5 werden von einem Kunststoffmaterial umschlossen, welches zu einem bandförmigen flachen Strang mit einer im wesentlichen glatten Seitenfläche 7 geformt ist. Dieses Flachband 6 wird anschließend zu einer Spirale geformt, derart, dass keine in-sich Torsion des Bandes 6 entsteht, d.h. die zumindest eine glatte Seitenfläche 7 immer die Innenseite der Spirale bildet.

Wie insbesondere aus der Fig. 2 zu entnehmen ist, werden die Kanäle 3 aus Rohren vorzugsweise aus einem Kunststoffmaterial gefertig, welche dann von einer weiteren Kunststoffhülle vorzugsweise einem Kunstharzschaum umgeben werden, welche zu dem Flachbandprofil geformt wird und die Rohre zu einer Einheit miteinander verbindet. Dabei sind beim ersten Ausführungsbeispiel die Kabel oder Fasern 5 jeweils einzeln oder in dünnen Faserbündeln (mit wenigen Fasern) zwischen den Rohren im Hüllenmaterial angeordnet. Durch diese Vereinzelung der Fasern/Kabel 5 wird die Flexibilität des Flachbands 6 erhöht und gleichzeitig die Homogenität von diesem verbessert.

Die so vorgeformte Flachbandspirale wird anschließend an den jeweiligen Längsrändern 8 verklebt oder verschweißt, wodurch sich der radial geschlossene Arbeitskanal 2 ausbildet, wie dies in der Fig. 3 dargestellt ist. Die Längsränder 8 können dabei entweder stoßgeschweißt/-verklebt werden oder sich geringfügig überlappen, wofür im letzteren Fall die Flachbandränder 8 im Überlappungsbereich gedünnt sind, um Materialverdickungen am fertigen Endoskopschaft 1 zu vermeiden.

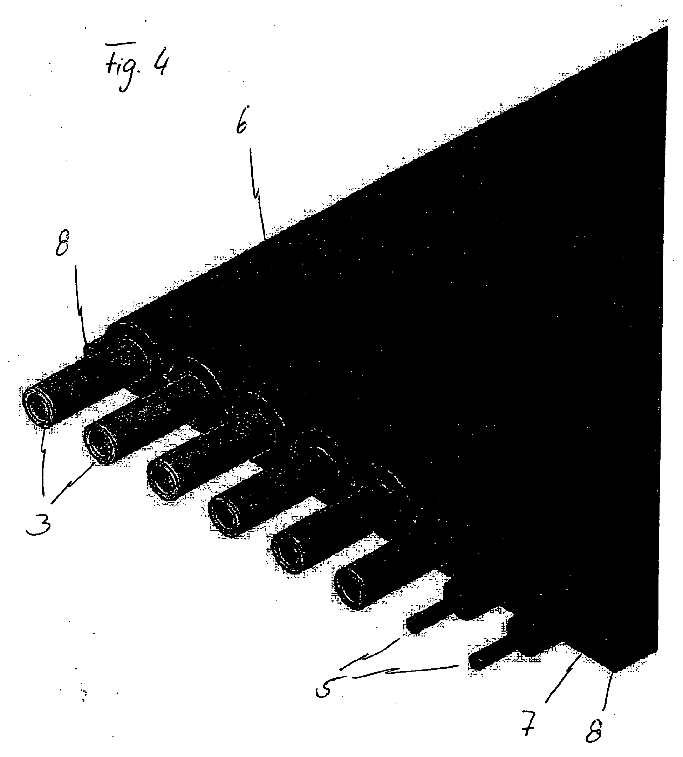

Ferner ist in der Fig. 4 ein zweites Ausführungsbeispiel der Erfindung ebenfalls in Form eines Flachbandes dargestellt, welches hinsichtlich seiner Form im wesentlichen dem ersten Ausführungsbeispiel entspricht, jedoch die Fasern/Kabel 5 nicht in Zwischenräumen zwischen den Kanälen 3 sondern als zu den Kanälen 3 separate Faser-/Kabelstränge an einer oder beiden Längsseiten des Flachbands angeordnet sind.

Grundsätzlich sind gemäß dem ersten wie auch zweiten Ausführungsbeispiel die Kanäle 3 durch Rohre gebildet und ggf. die Fasern/Kabel 5 mit einer Isolationsschicht umhüllt, wobei die so ausgebildeten Rohre und Fasern/Kabel in der das Flachband ausbildenden Hülle eingebettet sind. Bei beiden Ausführungsbeispielen ist es jedoch alternativ auch möglich, die Kanäle vergleichbar zur Ausführungsform gemäß Fig.1. durch Dorne auszubilden, welche von dem Hüllenmaterial umgossen und anschließend aus dem erhärteten Hüllenmaterial gezogen werden. Auch ist es grundsätzlich möglich, insbesondere auch im ersten und zweiten Ausführungsbeispiel, den Arbeitskanal 2 durch ein gesondertes schlauch- oder rohrförmiges Bauteil auszubilden, welches dann von der Flachbandspirale umgeben wird.

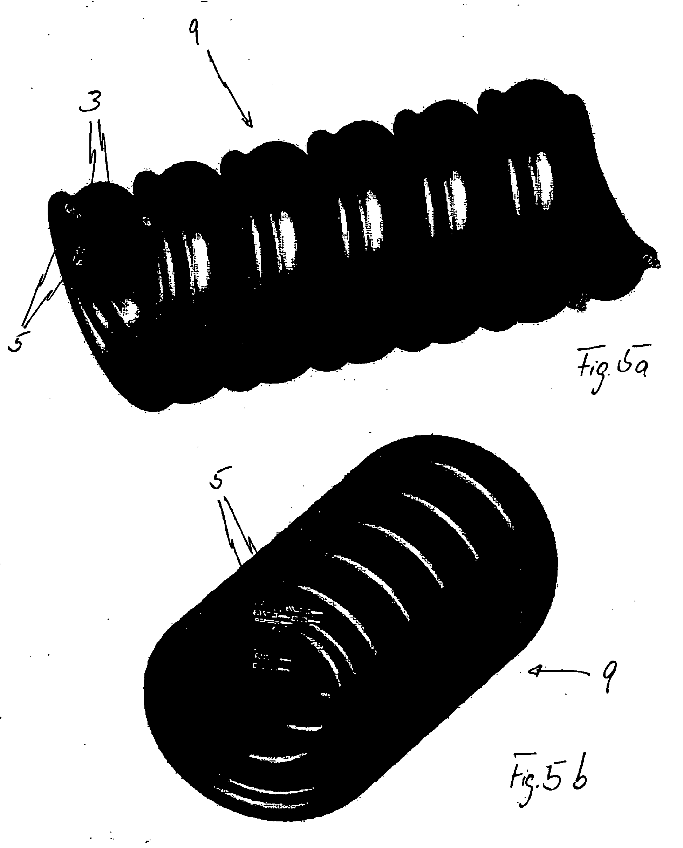

In der Fig. 5a, 5b ist ein drittes Ausführungsbeispiel für eine Versorgungs- und/oder Funktionskanal -Anordnung dargestellt.

Gemäß dem dritten Ausführungsbeispiel der Erfindung sind die Versorgungs- und/oder Funktionskanäle 3 radial zueinander zu einem Schlauchpaket 9 angeordnet, wobei die Kabel- oder Fasern 5 vorzugsweise in gleichen Winkelabständen zueinander an der Außenseiten des Schlauchpakets 9 liegen. Das Schlauchpaket 9 besteht aus einem extrudierten oder geschäumten Kunststoffstrang, der die Kanäle 3 selbst ausbildet, die vorliegend in ihrem Querschnitt zu Viertelkreisen geformt sind.

An dieser Stelle sei darauf hingewiesen, dass auch eine andere Anzahl von Versorgungs- und/oder Funktionskanälen vorgesehen sein kann als vier Kanäle gemäß dem dritten Ausführungsbeispiel. Auch wird das Schlauchpaket spiralförmig so um den Arbeitskanal geführt, wie dies in den ersten bis zweiten Ausführungsbeispielen dargestellt ist.

## Patentansprüche

1. Endoskopschaft (1) mit einem Arbeitskanal (2) und einer Anzahl von Versorgungs- und/oder Funktionskanälen (3), die so angeordnet sind, dass diese eine Überlänge bezüglich des Endoskopschafts (1) erhalten,
**dadurch gekennzeichnet, dass**
die Versorgungs- und/oder Funktionskanäle (3) zu einer Art Flachband (6) zusammengefasst sind, welches zu einer Flachbandspirale gewickelt oder wickelbar ist, die an ihren Längsrändern (8) zur Ausbildung des geschlossenen Arbeitskanals (2) verklebt ist.

2. Endoskopschaft nach Anspruch 1
**gekennzeichnet durch**
zumindest eine elektrische Leitung, eine Glasfaserleitung und/oder ein Leitungsbündel (5), die/das im wesentlichen parallel zu den Versorgungs- und/oder Funktionskanälen (3) angeordnet sind/ist.

3. Endoskopschaft nach Anspruch 2,
**durch gekennzeichnet, das**
die elektrische Leitung und/oder die Glasfaserleitung (5) zwischen den Versorgungs- und/oder Funktionskanälen (3) angeordnet ist/sind.

4. Endoskopschaft nach einem der vorstehenden Ansprüche,
**dadurch gekennzeichnet, dass**
die Versorgungs- und/oder Funktionskanäle (3) im Querschnitt eine Rechts- oder Dreiecksform aufweisen.

## Claims

1. An endoscope shaft (1) comprising a working conduit (2) and a number of supply and/or functional conduits (3), which are arranged so that they get an excessive length with respect to the endoscope shaft (1**), characterized in that** the supply and/or functional conduits (3) are combined into a kind of flat strip (6) which is wound or woundable into a flat spiral strip which are glued at their longitudinal edges (8) to form the sealed working conduit (2).

2. An endoscope shaft (1) according to claim 1, **characterized by** at least an electric line, a glass fiber line and/or a bundle of lines (5) which is/are arranged substantially in parallel to the supply and/or functional conduits (3).

3. An endoscope shaft (1 according to claim 2**, characterized in that** said electric line and/or said glass fiber line (5) is/are arranged between the supply and/or functional conduits (3).

4. An endoscope shaft (1) according to any one of the preceding claims, **characterized in that** the supply and/or functional conduits (3) have a rectangular or triangular shape in cross-section.

## Revendications

1. Tige endoscopique (1) comportant un canal de travail (2) et plusieurs canaux d'alimentation et/ou de fonction (3) disposés de telle manière qu'ils présentent une surlongueur par rapport à la tige endoscopique (1), **caractérisée en ce que** les canaux d'alimentation et/ou de fonction (3) sont réunis en une sorte de bande plate (6) qui est enroulée ou peut être enroulée de manière à former une spirale de bande plate collée sur ses bords longitudinaux (8) afin de former le canal de travail fermé (2).

2. Tige endoscopique selon la revendication 1, **caractérisée par** au moins un conducteur électrique, un conducteur à fibre de verre et/ou un faisceau de conducteurs (5) prévu en s'étendant sensiblement parallèlement aux canaux d'alimentation et/ou de fonction (3).

3. Tige endoscopique selon la revendication 2, **caractérisée en ce que** le conducteur électrique et/ou le conducteur à fibre de verre (5) est disposé entre les canaux d'alimentation et/ou de fonction (3).

4. Tige endoscopique selon l'une quelconque des revendications précédentes, **caractérisée en ce que** les canaux d'alimentation et/ou de fonction (3) présentent une section à forme rectangulaire ou triangulaire.
